# EUROPÄISCHE PATENTSCHRIFT

(11) **EP 2 292 300 B1**
(45) Veröffentlichungstag und Bekanntmachung des Hinweises auf die Patenterteilung: **08.05.2013**
(21) Anmeldenummer: 09011262.4
(22) Anmeldetag: 02.09.2009
(51) Int. Cl.: A61N 5/10, A61B 6/08, A61B 19/00

(54) **Vorrichtung und Verfahren zur Darstellung einer geometrischen Figur auf der Oberfläche eines Patientenkörpers**
Device and method for depicting a geometric figure on the surface of a patient's body
Dispositif et procédé de représentation d'une figure géométrique sur la surface d'un corps de patient

(43) Veröffentlichungstag der Anmeldung: 09.03.2011
(73) Patentinhaber: LAP GmbH Laser Applikationen, D-21337 Lüneburg (DE)
(72) Erfinder: Kindlein, Johann, DE - 21365 Adendorf (DE); Thurn, Tim, DE - 21335 Lüneberg (DE)
(74) Vertreter: Hauck Patent- und Rechtsanwälte

(56) Entgegenhaltungen:
- EP-A2- 2 098 169
- WO-A1-2009/011643
- US-B1- 6 314 311

## Beschreibung

Die Erfindung betrifft eine Vorrichtung und ein Verfahren zur Darstellung einer geometrischen Figur auf der Oberfläche eines auf einer Unterlage befindlichen Patientenkörpers und bezieht sich auf das Gebiet der Strahlungstherapie mit ionisierender Strahlung zur Krebsbehandlung. Dabei werden üblicherweise mehrere Strahlen aus unterschiedlichen Richtungen auf den zu behandelnden Körper gerichtet, so dass sie sich in einem Isozentrum schneiden. Dort wirkt die summierte Strahlendosis der verschiedenen auf das Isozentrum gerichteten ionisierenden Strahlen. Auf diese Weise wird die Beeinträchtigung des umliegenden Gewebes minimiert.

Als erster Schritt einer Strahlentherapie wird üblicherweise im Rahmen einer Bestrahlungsplanung eine Computertomographie-Aufnahme (CT) des auf einer Unterlage, wie einem Behandlungstisch, gelagerten und fixierten Patienten erstellt. Anhand dieser Aufnahme wird ein 3D-Modell des Patienten erstellt und der zu behandelnde Tumor lokalisiert und ein Bestrahlungsplan erstellt. Dies umfasst die Konturierung der Zielvolumina sowie die Berechnung der Dosis für die Bestrahlung und dabei insbesondere die Bestimmung der Anzahl und Lage der Strahlenfelder des Bestrahlungsgeräts, so dass der Tumor wie gewünscht bestrahlt wird.

Nach der Aufnahme des Patienten befindet sich der Patient nicht mehr auf der Unterlage. Außerdem findet im Rahmen einer anschließenden Strahlentherapie eine Vielzahl von Bestrahlungsterminen (üblicherweise bis zu 30 Fraktionen) statt. Daher ist wiederholt eine Übertragung der auf Grundlage des erstellten 3D-Modells des Patienten berechneten Strahlenfelder auf den realen Patienten erforderlich. Dieser Schritt wird auch als "Simulation" bezeichnet. Insbesondere muss der Patient für die Bestrahlung reproduzierbar so auf den Behandlungstisch gelegt werden, wie er bei der Erstellung der CT-Aufnahme lag.

Dazu ist es bekannt, eine Positionierung des Patienten mittels 3D-Röntgenaufnahmen ("Cone Beam") durchzuführen, wobei beispielsweise Knochen des Patienten die Referenzpunkte für eine Ausrichtung des Patienten bilden. Der Vorteil dieser Vorgehensweise ist eine hohe Genauigkeit. Außerdem müssen keine Referenzmarkierungen oder ähnliches auf der Patientenhaut aufgebracht werden. Der Nachteil dieser Vorgehensweise ist eine hohe Röntgenstrahlungsdosis für den Patienten, da die Röntgenpositionierung vor jeder Behandlungsfraktion erforderlich ist. Im Rahmen einer Strahlentherapie kann dies also üblicherweise bis zu 30 Mal erfolgen. Diese in einem großen Volumen auf den Körper applizierte Strahlendosis kann insbesondere bei jüngeren Patienten langfristig wieder einen neuen Krebs induzieren.

Gemäß einer bekannten alternativen Vorgehensweise kann der Patient anhand von drei Referenzpunkten auf seiner Haut, gebildet beispielsweise durch entsprechende Retroreflektoren, auf der Unterlage positioniert werden. Ein solches Verfahren ist beispielsweise bekannt aus DE 44 18 216 A1. Nach Durchführung der Bestrahlungsplanung werden die Schnittpunkte der Strahlenfelder mit der Hautoberfläche berechnet, die für die gewünschte Bestrahlung erforderlich sind. Die Schnittpunkte liegen dann in Form einer 3D-Koordinatentabelle vor. Nach der Neupositionierung des Patienten anhand der drei Referenzmarkierungen können dann die Koordinaten aus der Tabelle mit einem Lasersystem nacheinander auf der Körperoberfläche des Patienten dargestellt werden. Das Anfahren der Koordinaten kann beispielsweise durch eine Infrarot-Fernbedienung von einer Bedienperson gesteuert werden. Die von dem Laser jeweils dargestellten Punkte werden dann manuell, beispielsweise mit einem Stift, auf der Haut des Patienten eingezeichnet. Durch eine Verbindung der Punkte ergibt sich das gewünschte Schnittfeld für die Bestrahlung.

Ein solches Verfahren ist beispielsweise bekannt aus DE 44 21 315 A1 oder DE 195 24 951 A1. Die dazu eingesetzte Lasereinrichtung besteht insbesondere aus fünf motorisch beweglichen Lasern, wobei zwei in der Höhe verstellbare Laser eine horizontale Linie entlang des Behandlungstisches auf den Patienten richten, jeweils rechts und links von dem Behandlungstisch. Die übrigen drei Laser sind in einer Platte montiert, die sich über dem Behandlungstisch, beispielsweise einem CT-Tisch, befindet. Dabei ist ein Laser quer zur Tisch-Längsrichtung verschiebbar und richtet eine Linie entlang der Längsachse des Tisches auf den Patienten. Die beiden übrigen Laser in der Platte sind miteinander gekoppelt und richten eine gemeinsame Linie quer zur Längsachse des Tisches auf den Patienten. Durch die Kopplung zweier Laser können auch solche Koordinaten auf der Haut des Patienten dargestellt werden, die ansonsten unterhalb des Querdurchmessers des Patienten verschattet würden. Mit dem beschriebenen System ist es möglich, nahezu beliebige Koordinaten auf der Haut des Patienten anzufahren, wobei eine Koordinate jeweils durch ein Kreuz zweier Laserlinien angezeigt wird. Ein solches Lasersystem wird von der Anmelderin unter dem Namen "Dorado CT4" angeboten.

Die beschriebene Vorgehensweise ist allerdings verhältnismäßig zeitaufwendig und damit teuer, da Nutzungszeit in dem CT-Raum sehr teuer ist. Darüber hinaus ist das manuelle Einzeichnen der Punkte, insbesondere abhängig von der Adiposität des Patienten nicht immer ausreichend genau. Insbesondere können sich Hautmarkierungen verschieben. Daher finden in der Praxis nur teilweise Markierungen der Schnittfelder auf der Haut statt. Entsprechend ist die korrekte Bestrahlung des Patienten nicht immer ausreichend genau gewährleistet.

Es besteht außerdem ein Problem darin, dass die Position des Patienten zwischen der Bestrahlungsplanung und insbesondere einer dabei erfolgenden Aufnahme des Patientenkörpers einerseits und eines späteren Bestrahlungstermins andererseits trotz Verwendung gleicher Lagerungshilfen an dem Behandlungstisch zwangsläufig nicht immer identisch ist. Die oben erläuterten Vorgehensweisen zur reproduzierbaren Lagerung des Patienten sind dabei vergleichsweise aufwendig und erreichen in der Praxis nicht immer die erforderliche Genauigkeit.

Die Simulation ist nicht nur eine Übertragung des Bestrahlungsplans auf den Patienten, sondern auch ein wichtiges Element im Rahmen der Qualitätssicherung. Zum letzten Mal vor der Therapie wird der Bestrahlungsplan auf seine Plausibilität geprüft und festgestellt, ob die Strahlenfelder reproduzierbar auf das gewünschte Planungszielvolumen eingestellt werden können.

Ausgehend von dem erläuterten Stand der Technik liegt der Erfindung die Aufgabe zugrunde, eine Vorrichtung und ein Verfahren der eingangs genannten Art bereitzustellen, mit denen eine reproduzierbare präzise Lagerung des Patienten zuverlässig möglich ist.

Diese Aufgabe wird gemäß der Erfindung durch die Gegenstände der unabhängigen Ansprüche 1 und 9 gelöst. Vorteilhafte Ausgestaltungen finden sich in den abhängigen Ansprüchen sowie der Beschreibung und den Figuren.

Die Erfindung löst die Aufgabe zum einen durch eine Vorrichtung zur Darstellung einer geometrischen Figur auf der Oberfläche eines auf einer Unterlage befindlichen Patientenkörpers, umfassend:
- mindestens eine Projektionseinrichtung, mit der eine vorgegebene geometrische Figur auf die dreidimensionale Oberfläche des Patientenkörpers projizierbar ist,
- mindestens eine Steuereinrichtung und mindestens eine optische Sensoreinrichtung, mit der die auf die Oberfläche des Patientenkörpers projizierte geometrische Figur aufgenommen und die aufgenommenen Daten der Steuereinrichtung zugeführt werden können, wobei die Steuereinrichtung dazu ausgebildet ist, aus den von der Sensoreinrichtung aufgenommenen Daten die dreidimensionalen Koordinaten der auf den Patientenkörper projizierten geometrischen Figur zu ermitteln,
- und wobei die Steuereinrichtung dazu ausgebildet ist, die ermittelten dreidimensionalen Koordinaten der auf den Patientenkörper projizierten geometrischen Figur mit dreidimensionalen Sollkoordinaten zu vergleichen.

Zum anderen löst die Erfindung die Aufgabe durch ein Verfahren zur Darstellung einer geometrischen Figur auf der Oberfläche eines auf einer Unterlage befindlichen Patientenkörpers, umfassend die Schritte:
- eine vorgegebene geometrische Figur wird von einer Projektionseinrichtung auf die dreidimensionale Oberfläche des Patientenkörpers projiziert,
- die auf die Oberfläche des Patientenkörpers projizierte geometrische Figur wird mittels einer optischen Sensoreinrichtung aufgenommen und die aufgenommenen Daten werden einer Steuereinrichtung zugeführt, wobei die Steuereinrichtung aus den von der Sensoreinrichtung aufgenommenen Daten die dreidimensionalen Koordinaten der auf den Patientenkörper projizierten geometrischen Figur ermittelt,
- die ermittelten dreidimensionalen Koordinaten der auf den Patientenkörper projizierten geometrischen Figur werden von der Steuereinrichtung mit Sollkoordinaten verglichen.

Die vorgegebene geometrische Form kann dabei so gewählt sein, dass die vorgesehene Körperregion von der Strahlung in der im Rahmen der Bestrahlungsplanung gewünschten und berechneten Weise bestrahlt wird. Die geometrische Figur kann also ein Sollschnittfeld eines von einem Bestrahlungsgerät erzeugten Strahlenfeldes mit der Körperoberfläche des Patienten sein. Die Figur ist dann abhängig von der Form eines Multi-Leaf-Kollimators eines Bestrahlungsgeräts, beispielsweise eines Linearbeschleunigers und/oder der Position des Isozentrums eines zu behandelnden Tumors. Es können auch mehrere geometrische Formen dargestellt werden, sofern mehrere Strahlen für die Bestrahlung eingesetzt werden, die sich in einem in dem Körper des Patienten lokalisierten Isozentrum schneiden sollen.

Erfindungsgemäß wird eine bestimmte geometrische Figur vorgegeben, die dann mittels der Projektionseinrichtung auf die Oberfläche des Patienten projiziert wird. Die vorgegebene geometrische Form kann z. B. ein Kreis, ein Rechteck, ein Kreuz oder ähnliches sein. Die auf den realen Patientenkörper projizierte geometrische Figur wird von der Sensoreinrichtung aufgenommen. Dazu kann die Sensoreinrichtung eine oder mehrere Kameras besitzen, beispielsweise CCD- oder CMOS-Kameras. Bei der Verwendung mehrerer Kameras können die dreidimensionalen Koordinaten sicherer ermittelt werden, da die geometrische Figur von unterschiedlichen Blickwinkeln aus aufgenommen wird. Die Messdaten der Sensoreinrichtung werden der Steuereinrichtung zugeführt und diese ermittelt daraus in an sich bekannter Weise die dreidimensionalen Koordinaten der geometrischen Figur auf dem Patientenkörper. Die auf diese Weise ermittelten dreidimensionalen Koordinaten werden von der Steuereinrichtung dann mit zuvor bereitgestellten Sollkoordinaten der geometrischen Figur verglichen. Diese Sollkoordinaten sind diejenigen Koordinaten der geometrischen Figur auf der Oberfläche des auf der Unterlage befindlichen Patientenkörpers, die sich ergeben würden, wenn der Patient in einer zuvor festgelegten Referenzposition auf der Unterlage positioniert wäre und die geometrische Figur von der Projektionseinrichtung in gleicher Weise wie bei der Projektion auf den realen Patientenkörper auf den in der Referenzposition befindlichen Patientenkörper projiziert würde. Die Referenzposition ist die Position, die der Körper für die Bestrahlung einnehmen soll. Weichen die ermittelten Koordinaten von den Sollkoordinaten ab, befindet sich der Patientenkörper also nicht in der korrekten Position. Wie erläutert, wird der Patient mittels geeigneter Lagerungseinrichtungen bzw. Lagerungshilfen auf der Unterlage, die beispielsweise ein Behandlungstisch sein kann, positioniert. Wie ebenfalls bereits erläutert, kann jedoch auch bei Verwendung derselben Lagerungshilfen nicht gewährleistet werden, dass der Patient immer dieselbe Position einnimmt. Dies kann dann auf Grundlage des erfindungsgemäßen Vergleichs der Koordinaten in unten noch näher zu erläuternder Weise berücksichtigt bzw. korrigiert werden. Erfindungsgemäß wird also ein direkter Zusammenhang zwischen dem virtuellen Patienten im Computer, z.B. auf Grundlage eines CT-Bildes, und dem realen Patienten hergestellt

Die erfindungsgemäße Vorrichtung kann in dem Bestrahlungsraum mit dem Bestrahlungsgerät, beispielsweise einem Linearbeschleuniger ("LINAC"), in dem CT-Raum mit einem CT-Gerät oder in einem separaten Raum angeordnet sein. Entsprechend kann zu der Vorrichtung auch ein Bestrahlungsgerät oder ein CT vorgesehen sein. Insbesondere können die von der Steuereinrichtung bereitgestellten Sollkoordinaten der geometrische Figur in unten noch näher zu erläuternder Weise auf Grundlage einer CT-Aufnahme des Patienten erstellt werden. Darüber hinaus kann die Vorrichtung ein Computersystem mit einer graphischen Bilddarstellung und Software (Algorithmen) zur virtuellen Simulation einer Bestrahlung eines Patienten auf Grundlage von CT-Bildern mit Schnittstellen zur Übertragung von Bilddaten, Bestrahlungsdaten, Bestrahlungsfeldkonturen usw. aufweisen. Dieses Computersystem kann Teil der Steuereinrichtung sein. Die verschiedenen Einrichtungen der Vorrichtung können über ein lokales Netzwerk verbunden werden. Der Austausch der Daten kann in besonders einfacher Weise über den Digital Imaging and Communications in Medicine (DICOM und DICOM RT) Standard erfolgen. So kann das System unterschiedliche Funktionalitäten und Rechte in unterschiedlichen Räumen vergeben. Sämtliche Dateien können zur Qualitätskontrolle auf einer Server-Datei abgelegt werden.

Auch ist eine Markierung des Patienten mit einem Stift oder ähnlichem entlang der auf die Körperoberfläche projizierten geometrischen Figur möglich, jedoch nicht zwingend erforderlich. Wie erläutert, kann die geometrische Figur ein Sollschnittfeld eines von einem Bestrahlungsgerät erzeugten Strahlenfeldes mit der Körperoberfläche des Patienten sein. Auf Grundlage der Projektion kann das dargestellte Sollschnittfeld auf der Haut in besonders einfacher Weise markiert, beispielsweise mit einem Stift oder ähnlichem eingezeichnet werden. Die Vorrichtung muss dann nicht in dem Bestrahlungsraum angeordnet sein. Stattdessen kann die Simulation in einem separaten Raum erfolgen, dessen Benutzung kostengünstiger ist als die des Bestrahlungs- oder CT-Raums. Außerdem muss dann der Bestrahlungs- oder CT-Raum nicht durch den Einbau der erfindungsgemäßen Vorrichtung umgerüstet werden. Nach der Markierung des Patienten kann in dem Bestrahlungsraum mit dem Bestrahlungsgerät ein das Behandlungsfeld simulierendes Lichtfeld auf den Patienten gerichtet werden und der Patient so ausgerichtet werden, dass die Markierung und das Lichtfeld deckungsgleich sind. Dies kann manuell oder automatisch geschehen, wie weiter unten näher erläutert werden wird.

Es kann in besonders praxisgemäßer Weise vorgesehen sein, dass die Projektionseinrichtung mindestens eine Lichtquelle, insbesondere mindestens einen Laser, aufweist, wobei die geometrische Figur auf die Oberfläche des Patientenkörpers projizierbar ist, indem mindestens ein von der Lichtquelle erzeugter Lichtstrahl, insbesondere mindestens ein von dem Laser erzeugter Laserstrahl, ausreichend schnell entlang der Koordinaten geführt werden kann, so dass der Eindruck einer geschlossenen Kontur entlang der Koordinaten entsteht. Mittels eines Laserprojektors als optisches System werden also die Punkte der geometrischen Form nacheinander angefahren. Dabei werden die Punkte von dem Laserstrahl so schnell angefahren, dass für einen menschlichen Betrachter und auch eine Kamera eine vollständige Kontur entlang der Figur auf der Haut erscheint. Dabei ist kein zeitaufwendiges manuelles Anfahren von Koordinaten aus der Tabelle der Bestrahlungsplanung erforderlich. Der Einsatz gesundheitsgefährdender Röntgenstrahlung ist nicht erforderlich. Außerdem ist dann beispielsweise bei einer Anordnung der Vorrichtung in dem Bestrahlungsraum mit einem Bestrahlungsgerät keine manuelle Markierung der ein Sollschnittfeld des Bestrahlungsgeräts darstellenden geometrischen Figur auf der Haut des Patienten erforderlich. Vielmehr kann während der Projektion in einfacher Weise ein von dem Bestrahlungsgerät erzeugtes und die von dem Gerät erzeugte Strahlung anzeigendes Lichtschnittfeld in Übereinstimmung für die Bestrahlung mit dem projizierten Sollschnittfeld gebracht werden. In besonders einfacher Weise kann die Projektion erfolgen, wenn die Projektionseinrichtung mindestens zwei drehbare Spiegel aufweist, mit denen ein Laserstrahl auf die Oberfläche des Patientenkörpers reflektiert und entlang der Kontur der geometrischen Figur geführt werden kann. Die Spiegel können beispielsweise elektrisch angetriebene Galvanometerspiegel sein. Mit dieser Ausgestaltung wird eine besonders hohe Präzision bei der Projektion erreicht.

Damit das System funktionsfähig ist, muss eine Kalibrierung der Projektionseinrichtung durchgeführt werden, indem Kalibrierkoordinaten zur Verfügung gestellt werden, die mit der Steuereinrichtung angesteuert werden und somit die Kalibrierparameter bestimmt werden. Es müssen wenigstens sechs Punkte angesteuert werden. Einer der Kalibrierpunkte, der zur Kalibrierung verwendet wird, liegt dabei im Isozentrum einer CT-Anlage oder eines Linearbeschleunigers.

Die Sollkoordinaten können nach einer weiteren Ausgestaltung vorab im Rahmen einer Bestrahlungsplanung oder virtuellen Simulation ermittelt werden bzw. ermittelt worden sein, bei der eine virtuelle Darstellung zumindest der Oberfläche des Patientenkörpers erstellt wird. Als Sollkoordinaten werden dann diejenigen Koordinaten der geometrischen Figur vorgegeben, die sich ergeben würden, wenn die Projektionseinrichtung die geometrische Figur auf den virtuellen Patientenkörper projizieren würde. Im Rahmen der Bestrahlungsplanung kann beispielsweise eine CT-Aufnahme des Patienten erstellt werden. Daraus können ein virtueller Patientenkörper und insbesondere eine virtuelle Oberfläche des Patientenkörpers erstellt werden. Die dreidimensionalen Koordinaten des virtuellen Körpers sind bekannt, da die Anordnung der Aufnahmeeinrichtung, z. B. einer CT-Einrichtung, in dem jeweiligen Koordinatensystem bekannt ist. Die Position des Körpers bei der Bestrahlungsplanung dient dann als Referenzposition für die spätere Bestrahlung zur Krebsbehandlung. Die Projektionseinrichtung ist definiert angeordnet bzw. ausgerichtet, beispielsweise auf das Isozentrum eines Bestrahlungsgeräts kalibriert. Auch ist die relative Position der Projektionseinrichtung zu der Unterlage bekannt. Die bei der Projektion der geometrischen Figur auf den realen Patientenkörper fest definierte Anordnung bzw. Ausrichtung der Projektionseinrichtung wird ebenfalls der vorherigen Bestrahlungsplanung zugrunde gelegt. Insbesondere wird für die virtuelle Projektion der Figur auf den Patientenkörper dieselbe Anordnung der Projektionseinrichtung angenommen, wie sie bei der späteren realen Projektion auf den Patientenkörper vorliegt.

Es werden nun zumindest die 3D-Koordinaten der geometrischen Figur ermittelt, die sich ergeben würde, wenn die definiert angeordnete bzw. ausgerichtete Projektionseinrichtung die Figur auf den virtuellen Körper projizieren würde. Natürlich kann die geometrische Figur auch virtuell auf dem virtuellen Körper dargestellt werden. Der Patient wird zwar bei der Aufnahme des virtuellen Körpers mit den gleichen Lagerungshilfen auf dem Behandlungstisch positioniert wie bei der späteren Bestrahlung. Dennoch ergeben sich zwangsläufig Positionsabweichungen (gegebenenfalls auch durch körperliche Veränderungen wie Gewichtszu- oder - abnahme). Wird daher von der Projektionseinrichtung die geometrische Figur auf den realen Körper projiziert, ergibt sich eine Verformung der Figur und damit eine Abweichung der Koordinaten dieser Figur von denen der virtuellen Figur auf dem virtuellen Patientenkörper. Diese Abweichung wird erfindungsgemäß festgestellt und kann berücksichtigt, insbesondere korrigiert werden.

Es ist möglich, die geometrische Form bei dem virtuellen Patienten auf das Isozentrum eines Tumors (virtuell) zu projizieren. Dazu kann die erforderliche Ausrichtung der realen Projektionseinrichtung bestimmt und bei der späteren realen Projektion entsprechend eingestellt werden. Die virtuelle geometrische Form ist dann unverformt, beispielsweise ist ein Kreis also auch auf der Oberfläche des virtuellen Körpers ein Kreis. Anschließend kann der virtuelle Patient mit dem Isozentrum des Tumors virtuell in das Isozentrum eines bei einer anschließenden Therapie genutzten Bestrahlungsgeräts verschoben werden. Dabei verformt sich die geometrische Figur. Aus einem Kreis wird beispielsweise eine Ellipse oder ähnliches. Die Koordinaten dieser verformten Figur können dann als Sollkoordinaten genutzt werden, so dass auch der reale Patient mit dem Isozentrum des Tumors in das Isozentrum des Bestrahlungsgeräts verschoben wird. Erfindungsgemäß wird sichergestellt, dass einer Bestrahlung dieselbe Patientenposition zugrunde gelegt wird, wie sie bei der Bestrahlungsplanung und insbesondere der Aufnahme des virtuellen Patientenkörpers vorlag. Damit wird eine korrekte Bestrahlung erreicht.

Aus dem Vergleich der ermittelten Koordinaten mit den Sollkoordinaten kann von der Steuereinrichtung ein Verschiebungsvektor für die Unterlage errechnet werden, um die ermittelten Koordinaten mit den Sollkoordinaten in Übereinstimmung zu bringen. Die Unterlage kann weiterhin mittels geeigneter Verstellantriebe entlang einem oder mehrerer Freiheitsgrade bewegbar sein. Die Unterlage kann insbesondere entlang mindestens dreier Freiheitsgrade verschiebbar sein, die z. B. drei orthogonal zueinander ausgerichtete Achsen sein können. Eine Verstellbarkeit kann jedoch auch entlang von mehr als drei Freiheitsgraden, beispielsweise nach sechs Freiheitsgraden, möglich sein, so dass beispielsweise eine Rotation des Tisches möglich ist. Die Steuereinrichtung kann die Verstellantriebe dann so ansteuern, dass die Unterlage gemäß dem errechneten Verschiebungsvektor bewegt wird, der auf der Unterlage befindliche Patient also in die vorgegebene Referenzposition verschoben wird. Die Steuereinrichtung verfügt über einen entsprechenden Auswertealgorithmus, um die jeweiligen 3D-Koordinaten durch eine Verlagerung der Unterlage in Überlappung zu bringen ("Matching"). Die Parameter zur Verstellung der Unterlage können automatisch oder auf Eingabe einer Bedienperson auf die Verstellantriebe übertragen werden.

Die Vorrichtung kann eine Visualisierungssoftware aufweisen, mit der die Koordinaten der vorgegebenen geometrischen Figur und die sich daraus ergebende Figur sowie die Sollkoordinaten und die sich daraus ergebende Sollfigur darstellbar sind. Vorzugsweise sind sie in verschiedenen Ansichten darstellbar. Es können dann von einer Steuereinrichtung, jedoch vorzugsweise durch einen Benutzer der Vorrichtung, in besonders einfacher und komfortabler Weise auf Grundlage der Darstellung der Koordinaten der vorgegebenen geometrischen Figur und der sich daraus ergebenden Figur sowie der Sollkoordinaten und der sich daraus ergebenden Sollfigur mit der Visualisierungssoftware und durch ein vorzugsweise iteratives Bewegen der Unterlage beide Figuren in der Darstellung in Deckung gebracht werden.

Nach einer weiteren Ausgestaltung kann die Sensoreinrichtung mehrfach pro Sekunde eine Aufnahme der geometrischen Figur erstellen und die aufgenommenen Daten der Steuereinrichtung zuführen. Die Steuereinrichtung kann dann ebenfalls mehrfach pro Sekunde aus den aufgenommenen Daten die dreidimensionalen Koordinaten der auf den Patientenkörper projizierten geometrischen Figur ermitteln und mit den Sollkoordinaten vergleichen. Insbesondere können die von der Sensoreinrichtung aufgenommenen Daten in Echtzeit oder Nah-Echtzeit an die Steuereinrichtung weitergegeben und von dieser wiederum in Echtzeit oder Nah-Echtzeit ausgewertet werden. Durch diese Ausgestaltung ist je nach Anzahl der Messungen und Auswertungen pro Sekunde eine hochaufgelöste Bewegungskontrolle des Patienten möglich. Insbesondere kann beispielsweise bei einer Projektion der geometrischen Figur auf den Brustkorb des Patienten eine Kontrolle des Atmungszyklus erfolgen. Die Aufnahme und Auswertung der geometrischen Figur kann beispielsweise mehr als 10 Mal, vorzugsweise mehr als 20 Mal, pro Sekunde erfolgen. Entsprechend kann dann eine hochaufgelöste (Echtzeit- oder Nah-Echtzeit) Neupositionierung des Patienten abhängig von seiner Bewegung, beispielsweise einer Bewegung seines Brustkorbs, erfolgen, indem die Steuereinrichtung mehrfach pro Sekunde den Verschiebungsvektor errechnet und die Verstellantriebe der Unterlage so ansteuert, dass die Unterlage gemäß dem errechneten Verschiebungsvektor bewegt wird.

Die Vorrichtung kann weiterhin ein Bestrahlungsgerät zum Erzeugen ionisierender Strahlung für die Strahlentherapie aufweisen. Mit dem Bestrahlungsgerät kann eine der von dem Bestrahlungsgerät erzeugten Strahlung entsprechende Lichtstrahlung erzeugbar sein. Das Bestrahlungsgerät dient zur Tumorbehandlung und kann beispielsweise ein Linearbeschleuniger sein. Es ist dann möglich, dass die Steuereinrichtung das Bestrahlungsgerät mehrfach pro Sekunde immer dann zum Erzeugen der ionisierenden Strahlung ansteuert, wenn der Vergleich zwischen den ermittelten Koordinaten und den Sollkoordinaten einen Sollwert annimmt. Es kann bei dieser Ausgestaltung also ein sogenanntes "Gating" des Bestrahlungsgeräts erfolgen, indem dieses nur dann aktiviert wird, wenn der Vergleich den Sollwert ergibt oder diesem zumindest ausreichend nahe kommt. Es können selbstverständlich obere und untere Grenzwerte um einen bestimmten Wert vorgegeben werden, so dass ein Sollwertintervall vorliegt. Wenn der Vergleichswert in diesen Sollwertintervall fällt, wird das Bestrahlungsgerät aktiviert, anderenfalls deaktiviert. Auf diese Weise kann z. B. immer nur dann bestrahlt werden, wenn sich der Brustkorb und damit ein z. B. im Bereich des Thorax befindlicher Tumor in einer bestimmten Position des Atmungszyklus befindet. Es erfolgt also eine Nah-Echtzeit- oder Echtzeit-Steuerung des Bestrahlungsgeräts abhängig von der Patientenbewegung.

Erfindungsgemäß können mehrere Laserprojektionseinrichtungen, beispielsweise zwei, vorgesehen sein, um an sämtlichen Positionen des Patientenkörpers die geometrische Figur darstellen zu können. So kann beispielsweise jeweils mindestens ein links und rechts der den Patienten aufnehmenden Unterlage oberhalb der Unterlage angeordneter Projektor vorgesehen sein. Es ist jedoch auch möglich, dass die Projektionseinrichtung entlang einer im Wesentlichen kreisförmigen Bahn um eine den Patienten aufnehmende Unterlage bewegbar angeordnet ist. Insbesondere ist dann ein Laserprojektor ausreichend, der auf einer kreisförmigen Schiene oberhalb und/oder unterhalb der Unterlage verfahren werden kann, so dass er sämtliche Körperregionen eines Patienten erreichen und die Figur auf diesen darstellen kann. Die zur Darstellung einer Figur erforderlichen jeweiligen Haltepositionen des Projektors auf der Schiene können beispielsweise von der Steuereinrichtung oder dem jeweils eingesetzten virtuellen Simulationsprogramm bestimmt und eine Antriebseinrichtung zum Verfahren der Projektionseinrichtung entsprechend angesteuert werden.

Die erfindungsgemäße Vorrichtung kann insbesondere zur Durchführung des erfindungsgemäßen Verfahrens geeignet sein. Entsprechend kann das erfindungsgemäße Verfahren mit der erfindungsgemäßen Vorrichtung durchgerührt werden.

Ein Ausführungsbeispiel der Erfindung wird nachfolgend anhand einer Zeichnung näher erläutert. Es zeigen schematisch:
- Fig. 1: eine erfindungsgemäße Vorrichtung gemäß einer ersten Ausgestaltung,
- Fig. 2: eine erfindungsgemäße Vorrichtung gemäß einer zweiten Ausgestaltung,
- Fig. 3: eine erfindungsgemäße Vorrichtung gemäß einer dritten Ausgestaltung, und
- Fig. 4: eine schematische Darstellung zur Veranschaulichung der Funktionsweise der erfindungsgemäßen Vorrichtung.

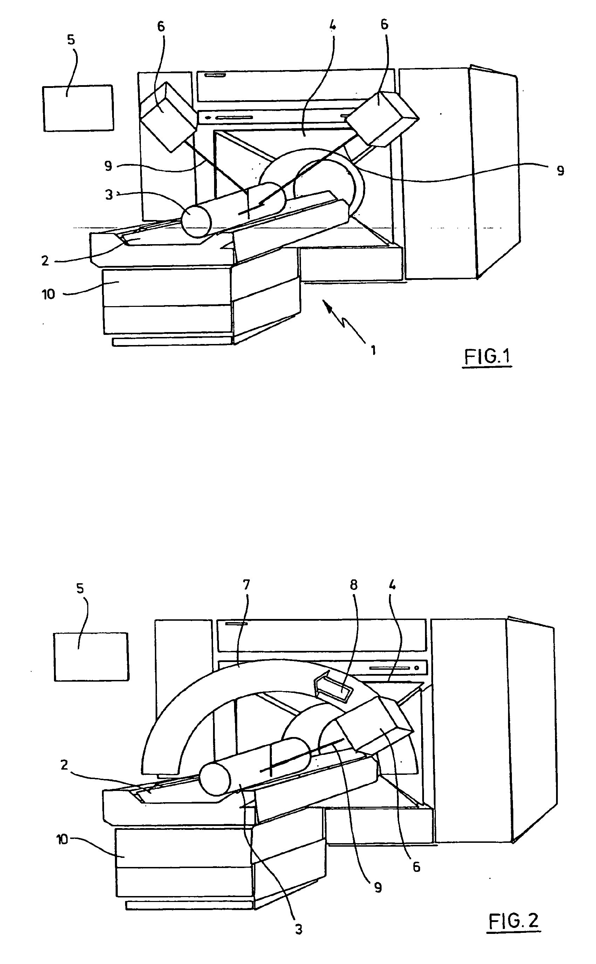

Soweit nichts anderes bestimmt ist, bezeichnen in den Figuren gleiche Bezugszeichen gleiche Gegenstände. In Fig. 1 ist eine erfindungsgemäße Vorrichtung 1 zur Darstellung einer geometrischen Figur auf der Oberfläche eines Patientenkörpers dargestellt. Die Vorrichtung weist eine mittels nicht näher dargestellter Verstellantriebe entlang mindestens dreier Achsen verstellbar gelagerte Unterlage 2, in dem Beispiel einen CT-Tisch 2, auf. Auf dem CT-Tisch 2 ist äußert schematisch ein Körper 3, beispielsweise ein Patientenkörper 3, dargestellt. Die Vorrichtung 1 umfasst weiterhin ein in einem Gehäuse angeordnetes Computertomographiegerät 4. In diesem CT-Gerät 4 können in an sich bekannter Weise CT-Aufnahmen und auf dieser Grundlage ein dreidimensionales Schnittbild eines Patienten erstellt werden. Anschließend findet im Rahmen einer Bestrahlungsplanung eine Ermittlung der Zielvolumina für die Bestrahlung mit ionisierender Strahlung statt. In einer Steuereinrichtung 5 werden auf Grundlage anhand des 3D-Schnittbildes des Patienten erstellter Zielvolumina beispielsweise die Koordinaten eines entsprechenden Sollschnittfelds eines für die Strahlentherapie vorgesehenen Therapiefeldes auf der Oberfläche des Patientenkörpers 3 berechnet.

Die in Fig. 1 dargestellte Vorrichtung umfasst zwei jeweils einen Laser aufweisende Projektionseinrichtungen 6. Die Projektionseinrichtungen 6 sind dabei ortsfest in dem die Vorrichtung aufnehmenden Raum angeordnet und dabei unter einem solchen Winkel zu dem Patientenkörper vorgesehen, dass sämtliche Regionen des Patientenkörpers 3 durch die Laser der Projektionseinrichtungen 6 gemeinsam erreicht werden können. Von mindestens einer der Projektionseinrichtungen 6 wird eine vorgegebene geometrische Figur auf die Oberfläche des Patientenkörpers 3 projiziert, indem ein von dem Laser erzeugter Laserstrahl 9 ausreichend schnell entlang der geometrischen Figur geführt wird, so dass für einen menschlichen Beobachter der Eindruck einer vollständigen Kontur entsteht. Der Laserstrahl 9 wird über zwei elektrisch angetriebene Galvanometerspiegel entlang der Figur geführt.

In Fig. 2 ist eine weitere Ausgestaltung der erfindungsgemäßen Vorrichtung dargestellt. Diese unterscheidet sich von der Vorrichtung aus Fig. 1 lediglich dadurch, dass anstelle zweier ortsfest angeordneter Projektionseinrichtungen 6 nur eine Projektionseinrichtung 6 vorgesehen ist, die mittels nicht näher dargestellter Verstellantriebe entlang einer oberhalb des Behandlungstisches 2 verlaufenden kreisförmigen Schiene 7 verfahren werden kann, wie durch den Pfeil 8 schematisch angedeutet. Die jeweilige Halteposition der Projektionseinrichtung 6 entlang der Schiene 7 kann dabei durch die Steuereinrichtung 5 in Abhängigkeit von der jeweils darzustellenden geometrischen Figur gewählt und die Verstellantriebe entsprechend angesteuert werden. Mit dieser Ausgestaltung ist nur eine Projektionseinrichtung 6 erforderlich, um auf der gesamten Oberfläche des Patientenkörpers 3 die geometrische Figur darstellen zu können.

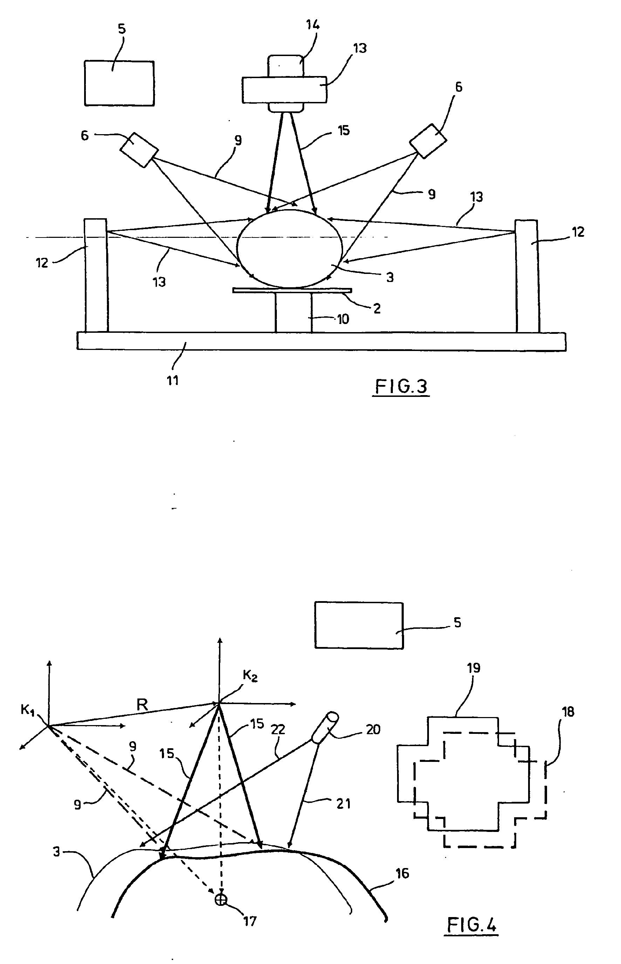

In Fig. 3 ist eine erfindungsgemäße Vorrichtung gemäß einer weiteren Ausgestaltung dargestellt. Gemäß dieser Ausgestaltung sind zwei ortsfest angeordnete Projektionseinrichtungen 6 zusammen mit der Steuereinrichtung 5 in dem für die Strahlentherapie genutzten Bestrahlungsraum angeordnet. Wiederum ist ein Patientenkörper 3 äußerst schematisch auf einer Unterlage 2 gezeigt, die über einen Sockel 10 auf dem Boden 11 des Behandlungsraums abgestützt ist. Die Vorrichtung in Fig. 3 weist weiterhin zwei ortsfest und auf beiden Seiten des Patientenkörpers 3 gegenüberliegend angeordnete Laser 12 auf. Diese dienen in an sich bekannter Weise zu einer Positionierung des Patientenkörpers 3 auf der Unterlage 2 anhand von an dem Patientenkörper 3 angeordneten Markierungen. Anschließend erfolgt eine Ansteuerung der Unterlage 2 so, dass der Patientenkörper 3 möglichst in die vorgegebene, während der Bestrahlungsplanung eingenommene Position bewegt wird.

Alternativ oder zusätzlich zu den Lasern 12 kann auch ein in Fig. 3 lediglich schematisch dargestelltes Laserscanning-System 13 oder ein anderes System zur Erfassung der Körperoberfläche vorgesehen sein. Ein solches System wird von der Anmelderin beispielsweise unter dem Namen "Galaxy" angeboten.

Die in Fig. 3 dargestellte Vorrichtung umfasst weiterhin ein Bestrahlungsgerät 14, vorliegend einen Linearbeschleuniger (LINAC). Das Bestrahlungsgerät 14 erzeugt eine ionisierende Strahlung zur Tumorbehandlung in dem Patientenkörper 3. Bei der Vorrichtung gemäß Fig. 3 wird wiederum von einer Projektionseinrichtung die vorgegebene geometrische Figur mit Laserstrahlung 9 auf den Patientenkörper 3 projiziert. Die Projektion der Figur erfolgt dabei wie bereits zu den Figuren 1 und 2 beschrieben. Gleichzeitig wird von dem Bestrahlungsgerät 14 eine der von dem Bestrahlungsgerät erzeugten ionisierenden Strahlung entsprechende Lichtstrahlung 15 erzeugt. Diese Lichtstrahlung 15 erzeugt auf dem Patientenkörper 3 ein Lichtschnittfeld. Die geometrische Figur kann ein Sollschnittfeld der Strahlung des Bestrahlungsgeräts 14 mit der Patientenoberfläche sein. In diesem Fall muss das dem Schnittfeld der von dem Bestrahlungsgerät 14 zu erzeugenden ionisierenden Strahlung mit der Patientenoberfläche entsprechende Lichtschnittfeld in Überlappung mit der geometrischen Figur gebracht werden. Anhand eines Vergleichs des von der Projektionseinrichtung 6 auf die Patientenkörperoberfläche projizierten Figur mit dem von dem Bestrahlungsgerät 14 erzeugten Lichtschnittfeld kann also die korrekte Ausrichtung des Patienten kontrolliert werden. Voraussetzung dafür ist jedoch, dass der Patient exakt wie bei der vorangegangenen CT-Aufnahme positioniert ist. Die Überprüfung dieser Positionierung soll im Folgenden näher erläutert werden.

Obgleich dies in den Fig. 1 bis 3 nicht dargestellt ist, weisen die dort gezeigten Vorrichtungen jeweils noch eine Sensoreinrichtung 20 auf, die beispielsweise in Fig. 4 gezeigt ist. Die Sensoreinrichtung 20 besitzt eine oder vorzugsweise mehrere Kameras, mit denen die von der bzw. den Projektionseinrichtungen 6 jeweils auf die Patientenoberfläche 3 projizierte geometrische Figur aufgenommen wird. Das Gesichtsfeld der Kamera(s) 20 wird durch die Pfeile 21, 22 schematisch dargestellt. Die aufgenommenen Daten werden von der Sensoreinrichtung 20 an die Steuereinrichtung 5 weitergegeben, die daraus die dreidimensionalen Koordinaten der auf den Patientenkörper 3 projizierten geometrischen Figur ermittelt. Vor der Projektion der geometrischen Figur auf den Patientenkörper 3 wurde mittels des beispielsweise in den Fig. 1 und 2 gezeigten CT-Geräts 4 eine dreidimensionale Aufnahme des Patientenkörpers 3 erstellt. Anhand dieser Aufnahme wurde dann die Bestrahlungsplanung für einen in dem Körper befindlichen Tumor vorgenommen. Insbesondere wurde der Tumor lokalisiert und es wurde mindestens ein Sollschnittfeld eines Bestrahlungsgeräts mit der Körperoberfläche ermittelt. Dieses Sollschnittfeld wurde dann als geometrische Figur vorgegeben. Mit der Steuereinrichtung 5 wurden dann auf dieser Grundlage und bei fest vorgegebener Anordnung der Projektionseinrichtung(en) 6 die 3D-Koordinaten dieser Figur als Sollschnittfeld auf der virtuellen Körperoberfläche berechnet. Diese Koordinaten wurden von der Steuereinrichtung 5 als Sollkoordinaten vorgegeben. Die Position des Patientenkörpers bei der CT-Aufnahme dient also als Referenzposition. Die von der Steuereinrichtung 5 ermittelten Koordinaten des real auf den Körper 3 projizierten Sollschnittfelds werden dann mit den Sollkoordinaten verglichen.

Diesbezüglich ist in Fig. 4 noch zu erkennen, dass für die Projektionseinrichtung 6 und das Bestrahlungsgerät 14 unterschiedliche Koordinatensysteme K1, K2, gültig sind. Ihre Koordinatenursprünge sind über einen Transformationsvektor R ineinander überführbar. Der reale Patientenkörper 3 ist in dem in Fig. 4 dargestellten Beispiel von der Referenzposition 16 des Patientenkörpers 3 entfernt. Das Isozentrum für die Bestrahlung ist für die Sollposition 16 des Patientenkörpers 3 bei 17 gezeigt. Aufgrund der Abweichung des realen Patientenkörpers 3 von der Referenzposition 16 sind das von der Projektionseinrichtung 6 auf den Patientenkörper 3 projizierte Sollschnittfeld 18 und das virtuelle Sollschnittfeld 19 auf dem virtuellen Patientenkörper nicht deckungsgleich, wie in Fig. 4 schematisch gezeigt. Zur Korrektur dieser Abweichung kann beispielsweise die Unterlage 2 bzw. ihre Verstellantriebe angesteuert werden, um den Patientenkörper 3 in die korrekte Position, nämlich die Referenzposition, zu bringen.

Es ist dabei auch möglich, dass von der Sensoreinrichtung 20 mehrfach pro Sekunde die auf den Patientenkörper 3 projizierte geometrische Figur aufgenommen wird und die entsprechenden Messdaten an die Steuereinrichtung 5 weitergegeben werden. Diese kann im Wesentlichen in Echtzeit wiederum mehrfach pro Sekunde daraus die 3D-Koordinaten der projizierten Figur bestimmen und diese jeweils mit den Sollkoordinaten vergleichen. Gerade wenn die geometrische Figur im Bereich des Brustkorbs auf den Patientenkörper projiziert wird, ist auf diese Weise eine Überwachung des Atmungszyklus des Patienten möglich. Entsprechend kann dann beispielsweise das Bestrahlungsgerät nur bei einer bestimmten Position des Brustkorbs, also einer bestimmten Stelle des Atmungszyklus, aktiviert und anderenfalls deaktiviert werden. Durch dieses Gating wird sichergestellt, dass auch bei Positionsänderungen der Organe und damit möglicherweise auch des Tumors durch eine Atmung des Patienten jederzeit die gewünschten Körperabschnitte bestrahlt werden.

## Patentansprüche

1. Vorrichtung zur Darstellung einer geometrischen Figur auf der Oberfläche eines auf einer Unterlage befindlichen Patientenkörpers, umfassend:
- mindestens eine Projektionseinrichtung (6), mit der eine vorgegebene geometrische Figur auf die dreidimensionale Oberfläche des Patientenkörpers (3) projizierbar ist,
- mindestens eine Steuereinrichtung (5) und mindestens eine optische Sensoreinrichtung (20), mit der die auf die Oberfläche des Patientenkörpers (3) projizierte geometrische Figur aufgenommen und die aufgenommenen Daten der Steuereinrichtung (5) zugeführt werden können, wobei die Steuereinrichtung (5) dazu ausgebildet ist, aus den von der Sensoreinrichtung (20) aufgenommenen Daten die dreidimensionalen Koordinaten der auf den Patientenkörper projizierten geometrischen Figur zu ermitteln,
- und wobei die Steuereinrichtung (5) dazu ausgebildet ist, die ermittelten dreidimensionalen Koordinaten der auf den Patientenkörper projizierten geometrischen Figur mit dreidimensionalen Sollkoordinaten zu vergleichen.

2. Vorrichtung nach Anspruch 1, **dadurch gekennzeichnet, dass** die Projektionseinrichtung (6) mindestens eine Lichtquelle, insbesondere mindestens einen Laser, aufweist, wobei die geometrische Figur auf die Oberfläche des Patientenkörpers (3) projizierbar ist, indem mindestens ein von der Lichtquelle erzeugter Lichtstrahl (9), insbesondere mindestens ein von dem Laser erzeugter Laserstrahl (9), ausreichend schnell entlang der Koordinaten geführt werden kann, so dass der Eindruck einer geschlossenen Kontur entlang der Koordinaten entsteht.

3. Vorrichtung nach einem der vorhergehenden Ansprüche, **dadurch gekennzeichnet, dass** die Sollkoordinaten im Rahmen einer Bestrahlungsplanung oder virtuellen Simulation ermittelt wurden, bei der eine virtuelle Darstellung zumindest der Oberfläche des Patientenkörpers (3) erstellt wurde, wobei die Sollkoordinaten diejenigen Koordinaten der geometrischen Figur sind, die sich ergeben würden, wenn die Projektionseinrichtung (6) die geometrische Figur auf den virtuellen Patientenkörper projizieren würde.

4. Vorrichtung nach einem der vorhergehenden Ansprüche, **dadurch gekennzeichnet, dass** die Vorrichtung eine Visualisierungssoftware aufweist, mit der die Koordinaten der vorgegebenen geometrischen Figur und die sich daraus ergebende Figur sowie die Sollkoordinaten und die sich daraus ergebende Sollfigur darstellbar sind, vorzugsweise in verschiedenen Ansichten darstellbar sind.

5. Vorrichtung nach einem der vorhergehenden Ansprüche, **dadurch gekennzeichnet, dass** mit der Steuereinrichtung (5) aus dem Vergleich der ermittelten Koordinaten mit den Sollkoordinaten ein Verschiebungsvektor für die Unterlage (2) errechnet werden kann, um die ermittelten Koordinaten mit den Sollkoordinaten in Übereinstimmung zu bringen.

6. Vorrichtung nach Anspruch 5, **dadurch gekennzeichnet, dass** die Unterlage (2) mittels geeigneter Verstellantriebe entlang einem oder mehrerer Freiheitsgrade bewegbar ist und die Steuereinrichtung (5) dazu ausgebildet ist, die Verstellantriebe so anzusteuern, dass die Unterlage (2) gemäß dem errechneten Verschiebungsvektor bewegt wird.

7. Vorrichtung nach einem der vorhergehenden Ansprüche, **dadurch gekennzeichnet, dass** die Sensoreinrichtung (20) dazu ausgebildet ist, mehrfach pro Sekunde eine Aufnahme von der geometrischen Figur zu erstellen und die aufgenommenen Daten der Steuereinrichtung (5) zuzuführen und, dass die Steuereinrichtung (5) dazu ausgebildet ist, ebenfalls mehrfach pro Sekunde aus den aufgenommenen Daten die dreidimensionalen Koordinaten der auf den Patientenkörper (3) projizierten geometrischen Figur zu ermitteln und mit den Sollkoordinaten zu vergleichen.

8. Vorrichtung nach den Ansprüchen 5 bis 7, **dadurch gekennzeichnet, dass** die Steuereinrichtung (5) dazu ausgebildet ist, mehrfach pro Sekunde den Verschiebungsvcktor zu errechnen und die Verstellantriebe so anzusteuern, dass dic Unterlage (2) gemäß dem errechneten Verschiebungsvektor bewegt wird.

9. Vorrichtung nach einem der Ansprüche 7 oder 8, **dadurch gekennzeichnet, dass** sie ein Bestrahlungsgerät (14) zum Erzeugen ionisierender Strahlung für eine Strahlentherapie des Patientenkörpers aufweist und, dass die Steuereinrichtung (5) dazu ausgebildet ist, das Bestrahlungsgerät (14) mehrfach pro Sekunde immer dann zum Erzeugen der ionisierenden Strahlung anzusteuern, wenn der Vergleich zwischen ermittelten Koordinaten und Sollkoordinaten einen Sollwert annimmt.

10. Verfahren zur Darstellung einer geometrischen Figur auf der Oberfläche eines auf einer Unterlage befindlichen Patientenkörpers, umfassend die Schritte:
- eine vorgegebene geometrische Figur wird von einer Projektionseinrichtung (6) auf die dreidimensionale Oberfläche des Patientenkörpers (3) projiziert,
- die auf die Oberfläche des Patientenkörpers (3) projizierte geometrische Figur wird mittels einer optischen Sensoreinrichtung (20) aufgenommen und die aufgenommenen Daten werden einer Steuereinrichtung (5) zugeführt, wobei die Steuereinrichtung (5) aus den von der Sensoreinrichtung (20) aufgenommenen Daten die dreidimensionalen Koordinaten der auf den Patientenkörper projizierten geometrischen Figur ermittelt,
- die ermittelten dreidimensionalen Koordinaten der auf den Patientenkörper projizierten geometrischen Figur werden von der Steuereinrichtung (5) mit Sollkoordinaten verglichen.

11. Verfahren nach Anspruch 10, **dadurch gekennzeichnet, dass** die geometrische Figur auf die Oberfläche des Patientenkörpers (3) projiziert wird, indem mindestens ein Lichtstrahl (9), insbesondere mindestens ein Laserstrahl (9), ausreichend schnell entlang der Koordinaten geführt wird, so dass der Eindruck einer geschlossenen Kontur entlang der Koordinaten entsteht.

12. Verfahren nach einem der Ansprüche 10 oder 11, **dadurch gekennzeichnet, dass** die Sollkoordinaten vorab im Rahmen einer Bestrahlungsplanung oder virtuellen Simulation ermittelt werden, bei der eine virtuelle Darstellung zumindest der Oberfläche des Patientenkörpers (3) erstellt wird, wobei als Sollkoordinaten diejenigen Koordinaten der geometrischen Figur vorgegeben werden, die sich ergeben würden, wenn die Projektionseinrichtung (6) die geometrische Figur auf den virtuellen Patientenkörper projizieren würde.

13. Verfahren nach einem der Ansprüche 10 bis 12, **dadurch gekennzeichnet, dass** die Koordinaten der vorgegebenen geometrischen Figur und die sich daraus ergebende Figur sowie die Sollkoordinaten und die sich daraus ergebende Sollfigur mit einer Visualisierungssoftware dargestellt werden, vorzugsweise in verschiedenen Ansichten dargestellt werden.

14. Verfahren nach Anspruch 13, **dadurch gekennzeichnet, dass** auf Grundlage der Darstellung der Koordinaten der vorgegebenen geometrischen Figur und der
sich daraus ergebenden Figur sowie der Sollkoordinaten und der sich daraus ergebenden Sollfigur mit der Visualisierungssoftware und durch insbesondere iteratives Bewegen der Unterlage beide Figuren in der Darstellung in Deckung gebracht werden.

15. Verfahren nach einem der Ansprüche 10 bis 14, **dadurch gekennzeichnet, dass** aus dem Vergleich der ermittelten Koordinaten mit den Sollkoordinaten ein Verschiebungsvektor für die Unterlage (2) errechnet wird, um die ermittelten Koordinaten mit den Sollkoordinaten in Übereinstimmung zu bringen.

16. Verfahren nach Anspruch 15, **dadurch gekennzeichnet, dass** die Unterlage (2) entlang einem oder mehrerer Freiheitsgrade bewegbar ist und die Steuereinrichtung (5) die Verstellantriebe so ansteuert, dass die Unterlage (2) gemäß dern errechneten Verschiebungsvektor bewegt wird.

17. Verfahren nach einem der Ansprüche 10 bis 16, **dadurch gekennzeichnet, dass** die Sensoreinrichtung (20) mehrfach pro Sekunde eine Aufnahme von der geometrischen Figur erstellt und die aufgenommenen Daten der Steuereinrichtung (5) zuführt und, dass die Steuereinrichtung (5) ebenfalls mehrfach pro Sekunde aus den aufgenommenen Daten die dreidimensionalen Koordinaten der auf den Patientenkörper (3) projizierten geometrischen Figur ermittelt und mit den Sollkoordinaten vergleicht.

18. Verfahren nach den Ansprüchen 15 bis 17, **dadurch gekennzeichnet, dass** die Steuereinrichtung (5) mehrfach pro Sekunde den Verschiebungsvektor errechnet und die Verstellantriebe so ansteuert, dass die Unterlage (2) gemäß dem errechneten Verschiebungsvektor bewegt wird.

## Claims

1. An apparatus for the representation of a geometrical figure on the surface of a patient's body that is situated on a support, comprising:
• at least one projection device (6), by which a given geometrical figure can be projected onto the three-dimensional surface of the patient's body (3),
• at least one control device (5) and at least one optical sensor device (20), by which the geometrical figure projected onto the surface of the patient's body (3) can be acquired and the acquired data can be supplied to the control device (5), wherein the control device (5) is realised to determine the three-dimensional coordinates of the geometrical figure projected onto the patient's body from the data acquired by the sensor device (20),
• and wherein the control device (5) is realised to compare the determined three-dimensional coordinates of the geometrical figure projected onto the patient's body with desired three-dimensional coordinates.

2. An apparatus according to claim 1, **characterised in that** the projection device (6) has at least one light source, at least one laser in particular, wherein the geometrical figure can be projected onto the surface of the patient's body (3) while at least one light beam (9) generated by the light source, at least one laser beam (9) generated by the laser in particular, can be guided along the coordinates sufficiently rapidly, so that the impression of a closed contour along the coordinates results.

3. An apparatus according to any one of the preceding claims, **characterised in that** the desired coordinates were determined in the frame of an irradiation planning or virtual simulation, in which a virtual representation of at least the surface of the patient's body (3) had been produced, wherein the desired coordinates are those coordinates of the geometrical figure that would result if the projection device (6) would project the geometrical figure onto the virtual patient's body.

4. An apparatus according to any one of the preceding claims, **characterised in that** the apparatus has a visualisation software, by which the coordinates of the given geometrical figure and the figure resulting from them as well as the desired coordinates and the desired figure resulting from them can be displayed, preferably displayed in different views.

5. An apparatus according to any one of the preceding claims, **characterised in that** from the comparison of the determined coordinates and the desired coordinates, a displacement vector for the support (2) can be calculated by the control device (5) in order to bring the determined coordinates into coincidence with the desired coordinates.

6. An apparatus according to claim 5, **characterised in that** the support (2) is movable along one or plural degrees of freedom by means of suitable adjustment drives, and the control device (5) is realised to control the adjustment drives such that the support (2) is moved according to the calculated displacement vector.

7. An apparatus according to any one of the preceding claims, **characterised in that** the sensor device (20) is realised to produce a mapping of the geometrical figure several times per second and to supply the acquired data to the control device (5), and that the control device (5) is realised to determine the three-dimensional coordinates of the geometrical figure projected onto the patient's body (3) from the mapping data several times per second and to compare them with the desired coordinates also several times per second.

8. An apparatus according to any one of claims 5 to 7, **characterised in that** the control device (5) is realised to calculate the displacement vector several times per second, and to control the adjustment drives such that the support (2) is moved according to the calculated displacement vector.

9. An apparatus according to any one of claims 7 or 8, **characterised in that** it has an irradiation machine (14) for generating ionizing radiation for a radiation therapy of the patient's body, and that the control device (5) is realised to control the irradiation machine (14) several times per second to generate the ionizing radiation always then when the comparison of determined coordinates and desired coordinates takes on a desired value.

10. A method for the representation of a geometrical figure on the surface of a patient's body that is situated on a support, comprising the steps:
• a given geometrical figure is projected onto the three-dimensional surface of the patient's body (3) by a projection device (6),
• the geometrical figure projected onto the surface of the patient's body (3) is acquired by means of an optical sensor device (20) and the acquired data are supplied to a control device (5), wherein the control device (5) determines the three-dimensional coordinates of the geometrical figure projected onto the patient's body from the data acquired by the sensor device (20),
• the determined three-dimensional coordinates of the geometrical figure projected onto the patient's body are compared with desired three-dimensional coordinates by the control device (5).

11. A method according to claim 10, **characterised in that** the geometrical figure is projected onto the surface of the patient's body (3) while at least one light beam (9), at least one laser beam (9) in particular, is guided along the coordinates sufficiently rapidly, so that the impression of a closed contour along the coordinates results.

12. A method according to any one of claims 10 or 11, **characterised in that** the desired coordinates are determined beforehand in the frame of an irradiation planning or virtual simulation, in which a virtual representation of at least the surface of the patient's body (3) is produced, wherein those coordinates of the geometrical figure are given as the desired coordinates that would result if the projection device (6) would project the geometrical figure onto the virtual patient's body.

13. A method according to any one of claims 10 to 12, **characterised in that** the coordinates of the given geometrical figure and the figure resulting from them as well as the desired coordinates and the desired figure resulting from them are displayed by a visualisation software, preferably displayed in different views.

14. A method according to claim 13, **characterised in that** based on the representation of the coordinates of the given geometrical figure and the figure resulting from the same, as well as on the desired coordinates and the desired figure resulting from the same, both figures are brought into coincidence in the representation by the visualisation software, and by iterative movement of the support in particular.

15. A method according to any one of claims 10 to 14, **characterised in that** from the comparison of the determined coordinates and the desired coordinates, a displacement vector for the support (2) is calculated in order to bring the determined coordinates into coincidence with the desired coordinates.

16. A method according to claim 15, **characterised in that** the support (2) is movable along one or plural degrees of freedom, and the control device (5) controls the adjustment drives such that the support (2) is moved according to the calculated displacement vector.

17. A method according to any one of claims 10 to 16, **characterised in that** the sensor device (20) produces a mapping of the geometrical figure several times per second and supplies the acquired data to the control device (5), and that the control device (5) determines the three-dimensional coordinates of the geometrical figure projected onto the patient's body (3) several times per second and compares them with the desired coordinates also several times per second.

18. A method according to any one of claims 15 to 17, **characterised in that** the control device (5) calculates the displacement vector several times per second, and controls the adjustment drives such that the support (2) is moved according to the calculated displacement vector.

## Revendications

1. Dispositif de représentation d'une figure géométrique sur la surface d'un corps de patient se trouvant sur un support, comprenant :
au moins un système de projection (6) qui permet de projeter une figure géométrique définie sur la surface tridimensionnelle du corps de patient (3),
au moins un système de commande (5) et au moins un système de capteur optique (20) qui permet d'enregistrer la figure géométrique projetée sur la surface du corps de patient (3) et de transmettre les données enregistrées au système de commande (5), le système de commande (5) étant configuré de manière à déterminer à partir des données enregistrées par le système de capteur (20) les coordonnées tridimensionnelles de la figure géométrique projetée sur le corps de patient,
et le système de commande (5) étant configuré de manière à comparer les coordonnées tridimensionnelles déterminées de la figure géométrique projetée sur le corps de patient aux coordonnées de consigne tridimensionnelles.

2. Dispositif selon la revendication 1, **caractérisé en ce que** le système de projection (6) comprend au moins une source lumineuse, en particulier au moins un laser, la figure géométrique pouvant être projetée sur la surface du corps de patient (3), en guidant suffisamment rapidement au moins un faisceau lumineux (9) produit par la source lumineuse, en particulier au moins un faisceau laser (9) produit par le laser, le long des coordonnées de manière à créer l'impression d'un contour fermé le long des coordonnées.

3. Dispositif selon une des revendications précédentes, **caractérisé en ce que** les coordonnées de consigne ont été déterminées dans le cadre d'un plan d'irradiation ou d'une simulation virtuelle, dans lequel une représentation virtuelle d'au moins la surface du corps de patient (3) a été établie, les coordonnées de consigne étant les coordonnées de la figure géométrique qui auraient été produites si le système de projection (6) avait projeté la figure géométrique sur le corps de patient virtuel.

4. Dispositif selon une des revendications précédentes, **caractérisé en ce que** le dispositif comporte un logiciel de visualisation qui permet de représenter les coordonnées de la figure géométrique définie et la figure en résultant ainsi que les coordonnées de consigne et la figure de consigne en résultant, de préférence dans différentes vues.

5. Dispositif selon une des revendications précédentes, **caractérisé en ce qu'**un vecteur de déplacement pour le support (2) peut être calculé avec le système de commande (5) à partir de la comparaison des coordonnées déterminées avec les coordonnées de consigne pour faire coïncider les coordonnées déterminées avec les coordonnées de consigne.

6. Dispositif selon la revendication 5, **caractérisé en ce que** le support (2) est mobile au moyen d'entraînements ajustables appropriés le long d'un ou de plusieurs degrés de liberté et le système de commande (5) est configuré de manière à commander les entraînements ajustables de telle sorte que le support (2) est déplacé selon le vecteur de déplacement calculé.

7. Dispositif selon une des revendications précédentes, **caractérisé en ce que** le système de capteur (20) est configuré de manière à établir plusieurs fois par seconde une prise de vue de la figure géométrique et à transmettre les données enregistrées au système de commande (5), et le système de commande (5) est configuré de manière à déterminer, également plusieurs fois par seconde à partir des données enregistrées, les coordonnées tridimensionnelles de la figure géométrique projetée sur le corps de patient (3) et à les comparer avec les coordonnées de consigne.

8. Dispositif selon les revendications 5 à 7, **caractérisé en ce que** le système de commande (5) est configuré de manière à calculer plusieurs fois par seconde le vecteur de déplacement et à commander les entraînements ajustables, de telle sorte que le support (2) est déplacé selon le vecteur de déplacement calculé.

9. Dispositif selon une des revendications 7 ou 8, **caractérisé en ce qu'**il comporte un appareil d'irradiation (14) pour produire un rayonnement ionisant pour une radiothérapie du corps de patient, et **en ce que** le système de commande (5) est configuré de manière à commander l'appareil d'irradiation (14) plusieurs fois par seconde toujours pour produire le rayonnement ionisant, si la comparaison entre les coordonnées déterminées et les coordonnées de consigne produit une valeur de consigne.

10. Procédé de représentation d'une figure géométrique sur la surface d'un corps de patient se trouvant sur un support, comprenant les étapes suivantes :
une figure géométrique définie est projetée par un système de projection (6) sur la surface tridimensionnelle du corps de patient (3),
la figure géométrique projetée sur la surface du corps de patient (3) est enregistrée au moyen d'un système de capteur optique (20) et les données enregistrées sont transmises à un système de commande (5), le système de commande (5) déterminant à partir des données enregistrées par le système de capteur (20) les coordonnées tridimensionnelles de la figure géométrique projetée sur le corps de patient,
les coordonnées tridimensionnelles déterminées de la figure géométrique projetée sur le corps de patient sont comparées par le système de commande (5) aux coordonnées de consigne.

11. Procédé selon la revendication 10, **caractérisé en ce que** la figure géométrique est projetée sur la surface du corps de patient (3), en guidant suffisamment rapidement au moins un faisceau lumineux (9), en particulier au moins un faisceau laser (9), le long des coordonnées de manière à créer l'impression d'un contour fermé le long des coordonnées.

12. Procédé selon une des revendications 10 ou 11, **caractérisé en ce que** les coordonnées de consigne sont déterminées préalablement dans le cadre d'un plan d'irradiation ou d'une simulation virtuelle, dans lequel une représentation virtuelle d'au moins la surface du corps de patient (3) a été établie, les coordonnées de la figure géométrique étant spécifiées comme coordonnées de consigne qui auraient été produites si le système de projection (6) avait projeté la figure géométrique sur le corps de patient virtuel.

13. Procédé selon une des revendications 10 à 12, **caractérisé en ce que** les coordonnées de la figure géométrique spécifiée et la figure en résultant ainsi que les coordonnées de consigne et la figure théorique en résultant sont représentées avec un logiciel de visualisation, sont représentées de préférence dans différentes vues.

14. Procédé selon la revendication 13, **caractérisé en ce que** sur la base de la représentation des coordonnées de la figure géométrique spécifiée et de la figure en résultant ainsi que des coordonnées de consigne et de la figure de consigne en résultant, les deux figures sont amenées en recouvrement dans la représentation avec le logiciel de visualisation et par déplacement en particulier itératif du support.

15. Procédé selon une des revendications 10 à 14, **caractérisé en ce qu'**un vecteur de déplacement pour le support (2) est calculé à partir de la comparaison des coordonnées déterminées avec les coordonnées de consigne pour faire coïncider les coordonnées déterminées avec les coordonnées de consigne.

16. Procédé selon la revendication 15, **caractérisé en ce que** le support (2) est mobile le long d'un ou de plusieurs degrés de liberté et le système de commande (5) commande les entraînements ajustables de telle sorte que le support (2) est déplacé selon le vecteur de déplacement calculé.

17. Procédé selon une des revendications 10 à 16, **caractérisé en ce que** le système de capteur (20) établit plusieurs fois par seconde une prise de vue de la figure géométrique et transmet les données enregistrées au système de commande (5), et **en ce que** le système de commande (5) détermine, également plusieurs fois par seconde à partir des données enregistrées, les coordonnées tridimensionnelles de la figure géométrique projetée sur le corps de patient (3) et les compare avec les coordonnées de consigne.

18. Procédé selon les revendications 15 à 17, **caractérisé en ce que** le système de commande (5) calcule plusieurs fois par seconde le vecteur de déplacement et commande les entraînements ajustables, de telle sorte que le support (2) est déplacé selon le vecteur de déplacement calculé.
